Europäisches Patentamt

European Patent Office

Office européen des brevets

(11) Veröffentlichungsnummer: **0 195 224**
**B1**

(12) **EUROPÄISCHE PATENTSCHRIFT**

(45) Veröffentlichungstag der Patentschrift: **26.09.90**

(51) Int. Cl.⁵: **A 61 K 6/08**, A 61 C 13/087

(21) Anmeldenummer: **86101568.3**

(22) Anmeldetag: **07.02.86**

(54) **Verfahren zur Herstellung von künstlichen Zähnen oder Zahnteilen und dafür geeigneter lagerfähiger Dentalwerkstoff.**

(30) Priorität: **21.02.85 DE 3506020**

(43) Veröffentlichungstag der Anmeldung:
**24.09.86 Patentblatt 86/39**

(45) Bekanntmachung des Hinweises auf die
Patenterteilung:
**26.09.90 Patentblatt 90/39**

(84) Benannte Vertragsstaaten:
**AT CH DE FR GB IT LI SE**

(56) Entgegenhaltungen:
**EP-A-0 041 676
AT-B- 338 436
AT-B- 375 545**

**POLYMER, Band 14, Nr. 1, Januar 1973, G.
ALLEN et al.: "Composites formed by interstitial
polymerization of vinyl monomers in
polyurethane elastomers: 1. Preparation and
mechanical properties of methyl methacrylate
based composites", Seiten 597-603**

(73) Patentinhaber: **Etablissement Dentaire IVOCLAR
FL-9494 Schaan (LI)**

(72) Erfinder: **Michl, Rudolf J., Dr.
Im Fetzer 49
FL-9494 Schaan (LI)**
Erfinder: **Wollwage, Peter
Auf Berg 113
FL-9493 Mauren (LI)**
Erfinder: **Zanghellini, Gerhard
Schaanerstrasse 80
FL-9490 Vaduz (LI)**

(74) Vertreter: **UEXKÜLL & STOLBERG
Patentanwälte
Beselerstrasse 4
D-2000 Hamburg 52 (DE)**

# EP 0 195 224 B1

**Beschreibung**

Die Erfindung betrifft einen neuartigen Dentalwerkstoff, der die Herstellung von künstlichen Zähnen und Zahnteilen in zwei Stufen ermöglicht.

Aus Polymer *14*, 597—603 (1973) ist es bekannt, schlagzähe Folien durch "Zwischenraum-Polymerisation" (interstitial polymerization) herzustellen. Dabei werden z.B. ein Polyol, ein Diisocyanat, ein Zinnkatalysator, Methylmethacrylat und ein Katalysator für dessen Heißpolymerisation zusammengemischt. Es entsteht ein Polyurethan-Gel, welches unter Einwirkung von Hitze ausgehärtet werden kann. Die Schlagzähigkeit wird dabei gegenüber reinem Polymethylmethacrylat (PMMA) verbessert.

In der Veröffentlichung findet sich kein Hinweis, solche Systeme zur Herstellung von Dentalwerkstoffen zu verwenden. Die angegebenen Zeiten für die Gelbildung von 4 bis 5 Stunden und die Polymerisationszeit zwischen 3 und 15 Stunden sind für einen brauchbaren Dentalwerkstoff auch völlig ungeeignet Weiterhin ist die Schlagzähigkeit für einen Dentalwerkstoff ein Parameter, der im allgemeinen nicht interessiert.

In der AT—B—338 436 werden flüssige photopolymerisierbare Zahnfüllmassen aus einem Polyurethanprepolymer mit endständigen ungesättigten Gruppen sowie einer damit polymerisierbaren monomeren Vinylverbindung und einem Photokatalysator beschrieben. Das Prepolymer enthält keine freien Isocyanat- oder Hydroxylgruppen mehr, so daß in der Zahnfüllmasse nur noch die photopolymerisierte radikalische Polymerisation der Vinylgruppen stattfinden kann.

Es wurde nunmehr überraschend gefunden, daß man durch Verwendung von Urethan/Methacrylat-Systemen mit mindestens zwei geeigneten Katalysatoren vorteilhafte Dentalwerkstoffe erhalten kann, die in Form von zwei Komponenten auch lagerstabil sind. Unter einem Dentalwerkstoff werden hier insbesondere Kronen- und Brückenmaterialien, Materialien zur Herstellung von künstlichen Zähnen und Inlays verstanden.

Gegenstand der Erfindung ist ein Dentalwerkstoff zur Herstellung von künstlichen Zähnen oder Zahnteilen wie Kronen oder Inlays aus zwei getrennten, bei Gebrauch miteinander zu vermischenden Komponenten, welcher dadurch gekennzeichnet ist, daß die erste Komponente

a) mindestens ein polyfunktionelles Polyisocyanat und die zweite Komponente

b) mindestens ein Polyol,

c) mindestens ein Methacrylatmonomer, welches mindestens zwei Hydroxylgruppen aufweist, und das auch als Komponente b) dienen kann,

d) einen Katalysator für die Heiß- oder Lichtpolymerisation des Methacrylatmonomers und

e) einen Katalysator für die Beschleunigung der Bildung eines Polyurethans aus den Bestandteilen a) und b), welcher die Polymerisation des Methacrylatmonomers nicht beschleunigt, enthält.

Das im Dentalwerkstoff gebildete Urethan kann z.B. aus der Reaktion von Polyolen mit Isocyanaten im geeigneten Molverhältnis entstehen. Beispiele für erfindungsgemäß verwendbare Polyole sind:

Ethylenglykol, Diethylenglykol, Triethylenglykol, 2,2-Bis-(4-hydroxyphenyl)propan, Bis-GMA, Propylenglykol, 1,3-Butandiol, 1,4-Butandiol, Cyclohexandiol, Bis-4,4'-(dihydroxyphenyl)-methan, Glycerin, Trimethylolethan, Trimethylolpropan, Pentaerythritol, Dipentaerythritol, 1,2,6-Hexantriol, Sorbitol, Polyethylenglykol, Polypropylenglykol, Polytetramethylenglykol; Polyester mit terminalen Hydroxygruppen, z.B. aus Adipinsäure und Diethylenglykol oder aus Maleinsäureanhydrid und Ethylenglykol; Polyhydroxyverbindungen mit Ethylenoxid oder Propylenoxid; teilweise veresterte Polyole, z.B: Glycerinmonomethacrylat und -acrylat, Pentaerythritolmonomethacrylat und -acrylat, Pentaerythritoldimethacrylat und -diacrylat; Copolymere aus Hydroxyethylmethacrylat und Methylmethacrylat. Die Polyole können einzeln oder als Gemisch verwendet werden. Es können auch Präpolymere von Di- oder Polyisocyanaten mit den obigen Polyolen eingesetzt werden.

Beispiele für erfindungsgemäß verwendbare Isocyanate sind:

Ethylen-diisocyanat, 1,6-Hexamethylendiisocyanat, 2,2,4- und 2,4,4-Trimethylhexamethylendiisocyanat, 1,6-Cyclohexandiisocyanat, Toluylendiisoyanat, Isophorondiisocyanat, Phorondiisocyanat, 1,5-Naphtylendiisocyanat, 1,3-Cyclopentylendiisocyanat, Phenylendiisocyanat, 2,4,6-Toluylentriisocyanat, 4,4'4''-Triphenylmethantriisocyanat, Xylylendiisocyanat, 3,3'-Diphenylmethandiisocyanat, 3,3'-Dimethyl-4,4'-diphenylmethandiisocyanat, 3,3'-Dimethylbiphenylendiisocyanat, 4,4'-Biphenylendiisocyanat, 1-Phenoxy-2,4'-phenylendiisocyanat, 1-tert.Butyl-2,4-phenylendiisocyanat, Methylen-bis-4,4'-cyclohexyl-diisocyanat, 1-Chlor-2,4-phenylendiisocyanat, 4,4'-Diphenyletherdiisocyanat, $\omega,\omega'$-Dipropyletherdiiso-cyanat, Dicyclohexylmethan-4,4'-diisocyanat und Tris(6-isocyanatohexyl)biuret.

Diese Isocyanate können einzeln für sich, als Gemisch untereinander oder als Präpolymere verwendet werde. Präpolymere werden durch Reaktion mit Di- oder Polyolen im Unterschuß erhalten. Es können auch Mischungen verschiedener Polyole mit verschiedenen Isocyanaten verwendet werden.

Die Bildung der Polyurethane erfolgt in Gegenwart an sich bekannter Katalysatoren, wie sie z.B. in der DE—OS 27 24 260 beschrieben sind. Insbesondere haben sich organische Zinnverbindungen als geeignet erwiesen, wobei Dibutylzinndiacetat besonders bevorzugt wird. Die Katalysatoren werden gewöhnlich in Mengen von 0,01 bis 10 Gew.%, bezogen auf die Gesamtmenge des Dentalwerkstoffs, eingesetzt. Bevorzugte Bereiche sind 0,2 bis 1 Gew.%, insbesondere 0,1 bis 0,6 Gew.%.

Der Dentalwerkstoff muß ferner polymerisierbare Vinylverbindungen enthalten. Hierbei handelt es sich um mindestens ein Methacrylatmonomer, welches mindestens zwei Hydroxylgruppen aufweist, vorzugsweise Bis-GMA (Bis-Phenol-A-Glycidylmethacrylat). Daneben können an sich bekannte Methacrylate in monofunktioneller oder polyfunktioneller Form allein oder in Mischungen eingesetzt werden. Biespiele sind Methylmethacrylat, Isobutylmethacrylat, Cyclohexylmethacrylat, Ethylenglycoldimethacrylat, Diethylenglycoldimethacrylat, Triethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat sowie die Reaktionsprodukte aus Isocyanaten, insbesondere Di- und/oder Triisocyanaten und OH-gruppenhaltigen Methacrylaten. Beispiele für letztere sind die Umsetzungsprodukte von 1 Mol Hexamethylendiisocyanat mit 2 Mol 2-Hydroxyethylmethacrylat, von 1 Mol Tris(6-isocyanatohexyl)biuret mit 3 Mol Hydroxyethylmethacrylat, und von 1 Mol Trimethylhexamethylendiisocyanat mit 2 Mol Hydroxyethylmethacrylat.

Der Anteil dieser meist langkettigen Verbindungen im Dentalwerkstoff bewegt sich im allgemeinen zwischen 5 und 95 Gew.%. Die Verbindungen mit mehreren OH-Gruppen, z.B. Bis-GMA dienen gleichzeitig als Alkoholkomponente für die Urethanreaktion.

Als Katalysatoren für die Heißpolymerisation der Methacrylate können die bekannten Peroxide wie Dibenzoylperoxid, Dilauroylperoxid, tert.-Butylperoctoat oder tert.-Butylperbenzoat eingesetzt werden, uber auch $\alpha,\alpha'$-Azodiisobutyronitril, $\alpha,\alpha$-Azo-bis-(isobutyroethylester), Benzpinakol und 2,2'-Dimethylbenzpinakol sind geeignet.

Es können aber auch Katalysatoren eingesetzt werden, die durch Strahlung aktivierbar sind. Die Verwendung von einem Photosensibilisator zusammen mit einem Reduktionsmittel wird bevorzugt. Beispiele für Photosensibilisatoren sind $\alpha$-Diketone wie 9,10-Phenanthrenchinon, Diacetyl, Furil, Anisil, 4,4'-Dichlorbenzil und 4,4'-Dialkoxybenzil. Campherchinon wird bevorzugt verwendet. Beispiele für Reduktionsmittel sind Amine wie Cyanoethylmethylanilin, Dimethylaminoethylmethacrylat, n-Butylamin, Triethylamin, Triethanolamin, N,N-Dimethylanilin oder N-Methyldiphenylamin. Eine besonders bevorzugte Kombination ist Campherchinon mit Cyanoethylmethylanilin.

Die erwähnten Katalysatoren werden im allgemeinen in Mengen von 0,01 bis 10 Gew.%, bezogen auf das polymerisierbare Material, verwendet, insbesondere in Mengen von 0,1 bis 5 Gew.%.

Es ist ferner zweckmäßig, dem Dentalwerkstoff sogenannte Trocknungsmittel beizufügen. Es handelt sich dabei vorzugsweise um Zeolithe, die in den gleichen Konzentrationen wie Füllstoffe eingesetzt werden können. Vorzugsweise beträgt der Gehalt an Zeolithen im Dentalwerkstoff etwa 2 bis 20, insbesondere 5 bis 12 Gew.%.

Je nach Verwendungszweck des Dentalwerkstoffs können noch andere anorganische Bestandteile vorhanden sein. Als Füllstoffe können z.B. Quarz, Kieselsäuren, insbesondere mikrofeine pyrogene, aber auch gefällte Kieselsäure, und die an sich bekannten Gläser Verwendung finden. Organische Füllstoffe, die ihrerseits wieder mit anorganischen Füllstoffen gefüllt sind, können ebenfalls eingesetzt werden. Die Füllstoffe werden im allgemeinen in Mengen von 5 bis 70, vorzugsweise 10 bis 50, insbesondere 15 bis 30 Gew.%, bezogen auf das Gesamtgewicht des Dentalwerkstoffs, verwendet.

Die anorganischen Bestandteile des Dentalwerkstoffs können silanisiert sein. Ein geeignetes Kupplungsmittel ist z.B. γ-Methacryloxypropyltrimethoxysilan. Die Menge des verwendeten Silans richtet sich nach der Art des eingesetzten Füllstoffs.

Dem Dentalwerkstoff können auch noch andere Komponenten, z.B. organische Polymere beigefügt werden. Vorzugsweise werden diese aus den vorher genannten polymerisierbaren Verbindungen hergestellt. Auch die Verwendung von Copolymeren ist möglich. Gegebenenfalls können auch die üblichen Pigmentierungsmittel wie z.B. Titandioxid enthalten sein.

Das gebildete Urethan verleiht dem Dentalwerkstoff innerhalb weniger Minuten eine gummiartige, elastische Konsistenz, die ein weiteres Bearbeiten z.B. durch Beschneiden ermöglicht. Die Konsistenz verändert sich nicht weiter, wenn als zweiter Katalysator ein solcher für die Heißpolymerisation verwendet wird. Voraussetzung ist dabei, daß die beiden Katalysatoren so ausgewählt werden, daß der Katalysator für die Urethanbildung die Polymerisation der Vinylverbindungen nicht beeinflußt. Dies könnte z.B. der Fall sein, wenn mann die Urethanreaktion mit einem Amin katalysieren würde. Bei Verwendung von Katalysatoren für die Kalthärtung oder die Härtung unter Licht hängt die Konsistenzänderung des Dentalwerkstoffs von der Art der eingesetzten Katalysatoren und deren Menge ab. Wenn es gewünscht wird, kann der Dentalwerkstoff nach Erreichen der gummielastischen Phase innerhalb weniger Minuten ausgehärtet werden. Ein kalthärtendes System kann aber auch lagerstabil in den Handel gebracht werden, wenn man z.B. die Komponenten getrennt lagert und erst vor Gebrauch zusammenmischt.

Vorzugsweise wird die Erfindung zur Herstellung eines provisorischen Kronen- und Brückenmaterials eingesetzt, aber auch für Inlays oder als Werkstoff verwendet werden. Für ein provisorisches Kronen- und Brückenmaterial werden im allgemeinen zwei Komponenten miteinander vermischt, wobei die eine Komponente das Isocyanat und die andere Komponente das Polyol und die ungesättigten Vinyl-Verbindungen enthält.

Ein provisorisches Kronen- und Brückenmaterial wird für ein Provisorium verwendet, d.h. es wird damit die Zeit überbrückt, bis die wirkliche Krone einsatzbereit ist. Bei der Herstellung einer Krone nimmt der Zahnarzt zunächst einen Silconabdruck von demjenigen unbehandelten Teil des Gebisses, das später

3

eine Krone bekommen soll. Nach Abdrucknahme wird der Zahn präpariert, d.h. konisch beschliffen. Auf diesem konischen Stumpf wird später die fertige Krone sitzen, die bis dahin von einer provisorischen abgedeckt werden muß.

Nach einem heute üblichen Verfahren wird der Siliconabdruck mit einem fließfähigen Material—z.B. einem Monomer/Polymergemisch oder einer Epiminverbindung—ausgegossen, und das Ganze wird auf den präparierten Zahn aufgesetzt. Die teigartige Phase des Materials geht für kurze Zeit in eine gummielastische über, worauf die Aushärtung relativ shcnell erfolgt. Während der kurzen gummielastischen Phase muß der Zahnarzt den Siliconabdruck abziehen, da im ausgehärteten Zustand bei Vorhandensein von Hinterschneidungen die Krone nicht mehr abgezogen werden kann. Dies ist ein relativ schwieriges Unterfangen, welches nicht immer gelingt. Wird der genaue Zeitpunkt verpaßt, härtet die provisorische Krone bereits aus. Dies bedeutet für den Zahnarzt, daß er die provisorische Krone auffräsen und mit dem Verfahren neu beginnen muß.

Mit dem erfindungsgemäßen Material wird zunächst ebenso verfahren, wobei jedoch die gummielastische Phase dauerhaft bestehen bleibt. Die elastische Krone kann somit immer abgezogen werden, ohne daß der Zahnarzt den genauen Zeitpunkt abpassen muß. Ferner ist die Krone in ihrer elastischen Phase durch einfaches Beschneiden völlig anpaßbar. Wenn der Zahnarzt die provisorische Krone in ihrem elastischen Zustand angepaßt hat, wird sie kalt oder heiß ausgehärtet und dann als Provisorium einzementiert.

Auch die Herstellung von Kunststoff-Inlays ist bisher ein sehr zeitraubender und mühsamer Vorgang. Soll ein Inlay eingesetzt werden, so nimmt der Zahnarzt einen Abdruck von der präparierten Kavität und stellt davon ein oder zwei Gipsmodelle her. Die Kavität im Gipsmodell wird mit Kunststoff gefüllt und dieser wird polymerisiert. Das so erhaltene Kunststoff-Inlay wird der Form entnommen und durch Beschleifen bzw. weiteres Bearbeiten in ein zweites Gipsmodell sorgfältig eingepaßt. Diese Arbeitsvorgänge werden nicht vom Zahnarzt selbst, sondern von Dental-Labors ausgeführt. Nachdem das Labor das Inlay fertiggestellt hat, wird es vom Zahnarzt in die Kavität des lebenden Zahnes einzementiert.

Das erfindungsgemäße Material läßt sich ebenfalls für die Inlay-Technik verwenden. Das Zwei-Komponenten-Material wird gemischt und in die präparierte Zahnkavität eingebracht, wo es gummielastisch aushärtet. Der Zahnarzt entnimmt der Kavität das gummielastische Inlay, paßt es sorgfältig ein und härtet es innerhalb weniger Minuten z.B. mit Licht aus. Das Inlay kann anschließend in die Kavität einzementiert werden. Die Arbeit des Dental-Labors mit den Gipsmodellen kann entfallen, es ist aber auch denkbar, daß die genaue Einpassung des Inlays und die Aushärtung durch das Labor vorgenommen werden.

Die Herstellung von künstlichen Zähnen erfolgt normalerweise so, daß man einen Teig aus Methylmethacrylat und Polymethylmethacrylat herstellt, daraus Ringe preßt, diese Ringe auf die Zahnform auflegt, preßt und polymerisiert. Bei hochwertigen Zähnen geschieht dies schichtweise, um dem Aussehen des natürlichen Zahnes so nahe wie möglich zu kommen. Das Pressen der Ringe geschieht mit teuren, viel Platz benötigenden, hydraulischen Pressen. Gemäß Erfindung kann man diese Ringe durch Gießen in eine Ringform erzeugen, in der die Urethanreaktion stattfindet. Man erhält in kurzer Zeit einen weichen Ring, ohne daß dafür kapitalintensive Geräte erforderlich sind.

Die Erfindung wird durch die nachfolgenden Beispiele erläutert. Alle Mengenangaben sind Gew.%.

Beispiele 1 bis 5 (Lichthärtende Materialien für provisorische Kronen)

Die Beispiele 1 bis 5 betreffen ein provisorisches Kronen- und Brückenmaterial. Es werden folgende Bestandteile zusammengemischt:

TABELLE 1

| | A | B | C | D | E |
|---|---|---|---|---|---|
| Bis-GMa | 68,0 | 61,2 | 34,0 | 61,2 | 34,0 |
| Butandioldimethacrylat | 17,0 | 15,3 | 8,5 | 15,3 | 8,5 |
| Decandioldimethacrylat | 2,64 | 2,38 | 1,32 | 2,38 | 1,32 |
| Zeolith (Natriumalumosilikat) | 10,0 | 9,0 | 5,0 | 9,0 | 5,0 |
| Polymer PP 1067* | — | 10,0 | 50,0 | — | — |
| Polymer PP 1068** | — | — | — | 10,0 | 50,0 |
| Dibutylzinndiacetat | 0,5 | 0,45 | 0,25 | 0,45 | 0,25 |
| Campherchinon | 0,36 | 0,32 | 0,18 | 0,32 | 0,18 |
| Cyanoethylmethylanilin | 1,50 | 1,35 | 0,75 | 1,35 | 0,75 |

\* PP 1067=Copolymer aus 10% HEMA und 90% MMA
\*\* PP 1068=Copolymer aus 20% HEMA und 80% MMA
    HEMA=Hydroxyethylmethacrylat
    MMA=Methylmethacrylat

Je 2 ml der Zusammensetzungen A bis E werden mit 1 ml Tris(6-isocyanatohexyl)biuret (Desmodur® N) gemischt. Die Polymerisation bei Raumtemperatur erfolgt in ca. 5 Minuten, man erhält eine elastische Polyurethanverbindung. Anschließend wird mit einer Halogenlampe 5 Minuten lang bestrahlt. Es bildet sich ein hartes Endprodukt, dessen physikalische Werte aus Tabelle 2 hervorgehen (Beispiele 1 bis 5).

TABELLE 2

| Physikalische Werte | Scutan | Protemp | Cronsin | Bsp. 1 | Bsp. 2 | Bsp. 3 | Bsp. 4 | Bsp. 5 |
|---|---|---|---|---|---|---|---|---|
| Druckfestigkeit N/mm$^2$ | 143±17 | 127±50 | 373±67 | 385 | 326±24 | 229±22 | 354±51 | 246±25 |
| E-Grenze N/mm$^2$ | 61±8 | 48±12 | 40±6 | 79 | 83±3 | 65±4 | 85±9 | 65±5 |
| Deformation bei 200 MPa% | — | — | 65±2 | 43 | 44±0,5 | 50±1 | 44±1 | 50±1 |
| Biegefestigkeit N/mm$^2$ | 51±18 | 59±5 | — | 81 | 75±7 | 56±9 | 56±8 | 71±2 |
| Biegemodul N/mm$^2$ | 860±400 | 1100±120 | — | 1150 | 2300±190 | 1800±250 | 1800±210 | 2200±140 |
| Randfaserdehnung % | 4,0±1,1 | 9,7±0,8 | — | 20 | — | 4,6±0,5 | — | 5,2±0,9 |

Wenn keine Meßwerte angegeben sind, waren diese nicht meßbar. Die Biegefestigkeit wird nach DIN 13922, die Druckfestigkeit nach DIN 13918 bestimmt. Die Bestimmung der anderen physikalischen Werte geht aus diesen DIN-Normen ebenfalls hervor.

Als Vergleich dazu werden marktgängige Materialien auf Epiminbasis (Scutan®), Bis-Acrylbasis (Protemp®) und PEMA-Basis (Cronsin®) mituntersucht. Außer dem entscheidenden Vorteil, daß die erfindungsgemäße provisorische Krone jederzeit abgezogen und in ihrem gummielastischen Zustand durch einfaches Beschneiden angepaßt werden kann, zeigen die Beispiele 1 bis 5 gegenüber Materialien des Standes der Technik auch hervorragende physikalische Eigenschaften.

Beispiele 6 bis 8 (Licht, kalt- und heißhärtende Dentalwerkstoffe)

Es werden die folgenden Komponenten zusammengemischt:

TABELLE 3

|  | F | G | H |
| --- | --- | --- | --- |
| Bis-GMA | 49,2 | 50,0 | 50,0 |
| Butandioldimethacrylat | 14,8 | 19,0 | 20,0 |
| Decandioldimethacrylat | 0,9 | — | — |
| Desmophen® 800* | 19,7 | 19,0 | 19,0 |
| Zeolith (Natriumalumosilikat) | 4,9 | 10,0 | 10,0 |
| Polymer PP 1067 (s. Tabelle 1) | 9,8 | — | — |
| Dibutylzinndiacetat | 0,6 | 0,5 | 0,5 |
| Campherchinon | 0,1 | — | — |
| Dimethyl-p-toluidin | — | — | 0,5 |
| Benzoylperoxid (50% in Weichmacher) | — | 1,5% | — |

*stark verzweigter hydroxylgruppenhaltiger Polyester aus Adipinsäure, Phthalsäure und einem Triol.

Man mischt folgende Bestandteile:

Beispiel 6:

2 Teile F+1 Teil Tris(6-isocyanatohexyl)biuret. Der Dentalwerkstoff wird 5 Minuten lang bei Raumtemperatur ausgehärtet. Anschließend wird 10 Minuten lang mit einem Lichtpolymerisationsgerät bestrahlt.

Beispiel 7:

2 Teile G+1 Teil Tris(6-isocyanatohexyl)biuret. Der Dentalwerkstoff wird 5 Minuten lang bei Raumtemperatur ausgehärtet (gummielastische Polyurethanphase). Anschließend wird während weiterer 5 Minuten bei 120°C in einem Druckpolymerisationsgerät bei 6 bar der Dentalwerkstoff unter Wasser auspolymerisiert.

Beispiel 8:

2 Teile H+1 Teil Tris(6-isocyanatohexyl)biuret+0,4 Teile 5%-ige Benzoylperoxidpaste in Cyclohexylphthalat. Der Dentalwerkstoff härtet kalt aus. Die Polyurethanreaktion findet in 5 Minuten statt. Der Dentalwerkstoff härtet bei Raumtemperatur innerhalb weiterer 10 Minuten fertig aus.

TABELLE 4

| | Beispiel | | |
|---|---|---|---|
| | 6 | 7 | 8 |
| Druckfestigkeit N/mm² | 400±35 | 459±38 | 352±43 |
| E-Grenze N/mm² | 73±5 | 111±4 | 81±5 |
| Deformation bei 200 MPa% | 49±1,0 | 29±1,0 | 39±1,0 |
| Biegefestigkeit N/mm² | 56±8 | 108±11 | 65±6 |
| Biegemodul N/mm² | 1730±330 | 3200±180 | 2100±160 |
| Randfaserdehnung % | 8,5±1,5 | 4,8±1,2 | 7,5±2,2 |

**Patentansprüche**

1. Lagerfähiger Dentalwerkstoff zur Herstellung von künstlichen Zähnen oder Zahnteilen wie Kronen oder Inlays aus zwei getrennten, bei Gebrauch miteinander zu vermischenden Komponenten, dadurch gekennzeichnet, daß die erste Komponente
a) mindestens ein polyfunktionelles Polyisocyanat und die zweite Komponente
b) mindestens ein Polyol,
c) mindestens ein Methacrylatmonomer, welches mindestens zwei Hydroxylgruppen aufweist, und das auch als Komponente b) dienen kann,
d) einen Katalysator für die Heiß- oder Lichtpolymerisation des Methacrylatmonomers und
e) einen Katalysator für die Beschleunigung der Bildung eines Polyurethans aus den Bestandteilen a) und b), welcher die Polymerisation des Methacrylatmonomers nicht beschleunigt, enthält.

2. Dentalwerkstoff nach Anspruch 1, dadurch gekennzeichnet, daß er—jeweils bezogen auf die polymerisierbaren organischen Bestandteile a), b) und c)—die Bestandteile a) und b) in eine Menge von 40 bis 95 Gew.% und den Bestandteil c) in einer Menge von 5 bis 60 Gew.% enthält.

3. Dentalwerkstoff nach den Ansprüchen 1 oder 2, dadurch gekennzeichnet, daß er als Bestandteile b) und c) mindestens ein Methacrylat, welches mindestens zwei Hydroxylgruppen aufweist, in einer Menge von 5 bis 95 Gew.% enthält.

4. Dentalwerkstoff nach einem der Ansprüche 1 bis 3, dadurch gekennzeichnet, daß er als Katalysator d) ein α-Diketon als Photosensibilisator zusammen mit einem Amin als Reduktionsmittel enthält.

5. Dentalwerkstoff nach einem der Ansprüche 1 bis 4 dadurch gekennzeichnet, daß er als Katalysator e) eine organische Zinnverbindung enthält.

6. Verwendung einer Masse, welche
a) mindestens ein polyfunktionelles Isocyanat,
b) mindestens ein Polyol,
c) mindestens ein Methacrylatmonomer, welches mindestens zwei Hydroxylgruppen aufweist, und das auch als Komponente b) dienen kann,
d) einen Katalysator für die Heiß- oder Lichtpolymerisation des Methacrylatmonomers und
e) einen Katalysator für die Beschleunigung der Bildung eines Polyurethans aus a) und b), welcher die Polymerisation des Methacrylatmonomers nicht beschleunigt,
enthält, zur Herstellung von künstlichen Zähnen oder Zahnteilen wie Kronen oder Inlays in einem zweistufigen Polymerisationsverfahren, bei dem man in einer ersten Stufe unter Formgebung durch Härtung bei Raumtemperatur einen elastischen Rohling herstellt und diesen, ggf. nach mechanischer Nachbearbeitung, in einer zweiten Stufe zu künstlichen Zähnen oder Zahnteilen aushärtet.

7. Verwendung nach Anspruch 6, dadurch gekennzeichnet, daß man eine Masse einsetzt, welche—jeweils bezogen auf die polymerisierbaren organischen Bestandteile a), b) und c) die Bestandteile a) und b) in einer Menge von 40 bis 95 Gew.% und den Bestandteil c) in einer Menge von 5 bis 60 Gew.% enthält.

8. Verwendung nach den Ansprüchen 6 oder 7, dadurch gekennzeichnet, daß man eine Masse verwendet, welche als Betandteile b) und c) mindestens ein Methacrylat, welches mindestens zwei Hydroxylgruppen aufweist, in einer Menge von 5 bis 95 Gew.% enthält.

9. Verwendung nach einem der Ansprüche 6 bis 8, dadurch gekennzeichnet, daß man eine Masse verwendet, welche als Katalysator d) ein α-Diketon als Photosensibilisator zusammen mit einem Amin als Reduktionsmittel enthält.

10. Verwendung nach einem der Ansprüche 6 bis 9, dadurch gekennzeichnet, daß man eine Masse verwendet, welche als Bestandteile a) und b) ein Prepolymer aus polyfunktionellen Isocyanaten und Polyolen im Unterschuß und c) das hydroxylgruppenhaltige Methacrylatmonomer enthält.

11. Verwendung nach einem der Ansprüche 6 bis 10, dadurch gekennzeichnet, daß man eine Masse einsetzt, welche als Katalysator e) eine organische Zinnverbindung enthält.

## Revendications

1. Matériau dentaire stockable pour la fabrication de dents artificielles ou de parties de dents artificielles telles que couronnes ou inlays, à partir de deux composants séparés à mélanger lors de l'emploi, caractérisé en ce que le premier composant renferme

a) au moins un polyisocyanate polyfonctionnel et le deuxième composant renferme

b) au moins un polyol,

c) au moins un méthacrylat monomère, qui possède au moins deux groupes hydroxyle et qui peut aussi servir de composant b),

d) un catalyseur pour la thermopolymérisation ou la photopolymérisation du méthacrylat monomère, et

e) un catalyseur pour l'accélération de la formation d'un polyuréthane à partir des constituants a) et b), qui n'accélère pas la polymérisation du méthacrylate monomère.

2. Matériau dentaire selon la revendication 1, caractérisé en ce qu'il contient les constituants a) et b) en une proportion de 40 à 95% en poids et le constituant c) en une proportion de 5 à 60% en poids, chaque fois par rapport aux constituants organiques polymérisables a), b) et c).

3. Matériau dentaire selon la revendication 1 ou 2, caractérisé en ce qu'il contient, comme constituants b) et c), au moins un méthacrylat, qui renferme au moins deux groupes hydroxyle, en une proportion de 5 à 95% en poids.

4. Matériau dentaire selon l'une des revendications 1 à 3, caractérisé en ce qu'il contient, comme catalyseur d), une α-dicétone en tant que photosensibilisateur en même temps qu'une amine comme réducteur.

5. Matériau dentaire selon l'une des revendications 1 à 4, caractérisé en ce qu'il contient, comme catalyseur e), un composé organostannique.

6. Utilisation d'une masse, qui renferme

a) au moins un isocyanate polyfonctionnel,

b) au moins un polyol,

c) au moins un méthacrylate monomère, qui possède au moins deux groupes hydroxyle et qui peut aussi servir de composant b),

d) un catalyseur pour la thermopolymérisation ou la photopolymérisation du méthacrylate monomère, et

e) un catalyseur pour l'accélération de la formation d'un polyuréthane à partir de a) et b), qui n'accélère pas la polymérisation du méthacrylate monomère,

pour la fabrication de dents ou parties de dents artificielles, telles que des couronnes ou des inlays, par un procédé en deux stades, selon lequel on fabrique, au cours d'un premier stade, une ébauche élastique par moulage par durcissement à température ambiante, et, dans un deuxième stade, on durcit cette ébauche sous forme de dent ou partie de dent artificielle, éventuellement après retraitement mécanique.

7. Utilisation selon la revendication 6, caractérisée en ce que l'on utilise une masse qui renferme les constituants a) et b) en une proportion de 40 à 95% en poids et le constituant c) en une proportion de 5 à 60% en poids, chaque fois par rapport aux constituants organiques polymérisables a), b) et c).

8. Utilisation selon la revendication 6 ou 7, caractérisée en ce que l'on utilise une masse qui renferme, comme constituants b) et c), au moins un méthacrylate, possédant au moins deux groupes hydroxyle, en une proportion de 5 à 95% en poids.

9. Utilisation selon l'une des revendications 6 à 8, caractérisée en ce que l'on utilise une masse qui renferme, comme catalyseur d), une α-dicétone en tant que photosensibilisateur en même temps qu'une amine comme réducteur.

10. Utilisation selon l'une des revendications 6 à 9, caractérisée en ce que l'on utilise une masse qui renferme, comme constituants a) et b) un prépolymère d'isocyanates polyfonctionnels et de polyols en défaut et c) un méthacrylat monomère comportant des groupes hydroxyle.

11. Utilisation selon l'une des revendications 6 à 10, caractérisée en ce que l'on met en oeuvre une masse qui renferme, comme catalyseur e), un composé organostannique.

## Claims

1. Storable dental material for the production of false teeth or parts thereof, such as crowns or inlays, comprised of two separate components to be mixed together at the time of use, characterized in that the first component contains

a) at least one polyfunctional polyisocyanate and the second component contains

b) at least one polyol,

c) at least one methacrylate monomer, which has at least two hydroxyl groups, and which can also serve as component b),

d) a catalyst for the hot or light polymerization of the methacrylate monomer and

e) a catalyst for accelerating the formation of a polyurethane from components a) and b) which does not accelerate the polymerization of the methacrylate monomer.

2. A dental material according to claim 1, characterized in that it contains the components a) and b) in a quantity of 40 to 95% by weight and the component c) in a quantity of 5 to 60% by weight,—based in each case on the polymerizable organic components a), b) and c).

3. A dental material according to claim 1 or 2, characterized in that as components b) and c) it contains at least one methacrylate, which has at least two hydroxyl groups, in a quantity of 5 to 90% by weight.

4. A dental material according to one of claims 1 to 3, characterized in that as catalyst d) it contains an alpha-diketone as a photo-sensitizer together with an amine as a reducing agent.

5. A dental material according to one of claims 1 to 4, characterized in that it contains an organic tin compound as catalyst e).

6. Use of a mass which contains

a) at least one polyfunctional isocyanate,

b) at least one polyol,

c) at least one methacrylate monomer, which has at least two hydroxyl groups, and which can also serve as component b),

d) a catalyst for the hot or light polymerization of the methacrylate monomer and

e) a catalyst for the acceleration of the formation of a polyurethane from a) and b) which does not accelerate the polymerization of the methacrylate monomer,

for producing false teeth or parts thereof, such as crowns or inlays, in a two-stage polymerization process, in which in a first stage an elastic blank is produced by hardening at ambient temperature accompanied by shaping and in a second stage, optionally after mechanical finishing, the latter is cured to form false teeth or parts thereof.

7. Use according to claim 6, characterized in that a mass is used which contains the components a) and b) in a quantity of 40 to 95% by weight and the component c) in a quantity of 5 to 60% by weight—based in each case on the polymerizable organic components a), b) and c).

8. Use according to claims 6 or 7, characterized in that a mass is used which as components b) and c) contains at least one methacrylate, which has at least two hydroxyl groups, in a quantity of 5 to 95% by weight.

9. Use according to one of claims 6 to 8, characterized in that a mass is used which contains as catalyst d) an alpha-diketone as a photo-sensitizer together with an amine as a reducing agent.

10. Use according to one of claims 6 to 9, characterized in that a mass is used which contains as components a) and b) a prepolymer of polyfunctional isocyanates and polyols in excess and as component c) the methacrylate monomer which contains the hydroxyl groups.

11. Use according to claims 6 to 10, characterized in that a mass is used which contains an organic tin compound as catalyst e).